(19) Europäisches Patentamt
European Patent Office
Office européen des brevets

(11) **EP 0 733 590 B1**

## (12) FASCICULE DE BREVET EUROPEEN

(45) Date de publication et mention
de la délivrance du brevet:
**19.12.2001 Bulletin 2001/51**

(51) Int Cl.[7]: **C01D 3/22**, C01D 3/24,
C01D 3/26

(21) Numéro de dépôt: **96200623.5**

(22) Date de dépôt: **07.03.1996**

(54) **Sel comprenant plus de 99,5 % en poids de chlorure de sodium et son utilisation**

Salz, das 99,5 Gewichtsprozent Natriumchlorid enthält und dessen Verwendung

Salt having more than 99,5% by weight sodium chloride and its use

(84) Etats contractants désignés:
**AT BE CH DE DK ES FI FR GB GR IE IT LI LU NL
PT SE**

(30) Priorité: **24.03.1995 FR 9503588**

(43) Date de publication de la demande:
**25.09.1996 Bulletin 1996/39**

(73) Titulaire: **SOLVAY (Société Anonyme)
1050 Bruxelles (BE)**

(72) Inventeur: **Ninane, Léon
F-54110 Dombasle-sur-Meurthe (FR)**

(74) Mandataire: **Dufrasne, Eugène et al
Solvay S.A., Département Propriété Industrielle,
310, rue de Ransbeek
1120 Bruxelles (BE)**

(56) Documents cités:
**EP-A- 0 352 847          FR-A- 2 562 884**

• **DE INGENIEUR, vol. 84, no. 45, 10 Novembre
1972, NL, pages 53-56, XP002006549 J. F. WITTE:
"Continue produktie van grove
keukenzoutkristallen"**
• **DATABASE WPI Week 8929 Derwent
Publications Ltd., London, GB; AN 209381
XP002006550 & JP-A-01 145 320 (JAPAN
TOBACCO & SALT PUB) , 7 Juin 1989**
• **Ullmanns Encyclopädie der technischen
Chemie, 4ème Ed. Vol.17, 1979, Natriumchlorid**
• **Catalogue Merck 1987/88, Natriumchlorid**

Remarques:
Le dossier contient des informations techniques
présentées postérieurement au dépôt de la
demande et ne figurant pas dans le présent
fascicule.

## Description

L'invention concerne du sel comprenant plus de 99,5 % en poids de chlorure de sodium, notamment du sel de qualité pharmaceutique.

Le sel de qualité pharmaceutique doit répondre à des normes de pureté sévères, imposées par des directives nationales ou internationales. Le sel destiné à certaines applications industrielles est également soumis à des normes rigoureuses de pureté. Ainsi, la norme NF T90-612 concernant le sel pour la régénération des résines échangeuses d'ions impose que celui-ci contienne au moins 99,5 % en poids de chlorure de sodium.

Ces exigences sévères concernant la pureté du sel pharmaceutique ou industriel a pour résultat qu'il est impossible d'y incorporer une quantité effective de substance antiagglutinante. Il s'ensuit que ce type de sel présente en général la propriété désavantageuse de prendre rapidement en masse, ce qui rend sa conservation problématique et son utilisation difficile.

L'invention remédie à cet inconvénient, en fournissant une variété nouvelle de sel, qui respecte les exigences sévères de pureté exposées plus haut, sans présenter de tendance exagérée à l'agglutination.

En conséquence, l'invention concerne du sel comprenant plus de 99,5 % en poids de chlorure de sodium et se caractérisant en ce qu'il se trouve à l'état de granules sphéroïdaux de diamètre moyen supérieur à 0,6 mm et inférieur à 3 mm, et présente une teneur pondérale en eau inférieure à 0,5%.

On entend désigner par granule sphéroïdal, une particule solide de sel qui est essentiellement délimitée par des surfaces courbes et convexes, qui est sensiblement démunie d'arêtes vives et qui s'inscrit à l'intérieur d'un tétraèdre régulier. Les granules sphéroïdaux du sel selon l'invention ont généralement un profil sensiblement de révolution, qui peut varier de la lentille à la sphère idéale. Ils présentent avantageusement un rapport axial moyen au moins égal à 0,5, défini par la relation :

$$\frac{\Sigma^{i=n} a_i : b_i}{n}$$

où $a_i$ et $b_i$ désignent respectivement la plus petite et la plus grande dimension axiale d'un granule sphéroïdal, et n désigne le nombre de granules sphéroïdaux d'un échantillon de granules sphéroïdaux. Dans le cas idéal, les granules constituant le sel selon l'invention sont des sphères.

Le diamètre moyen des granules sphéroïdaux du sel selon l'invention est défini par la relation :

$$\frac{\Sigma \, n_i d_i}{\Sigma \, n_i}$$

où $n_i$ désigne la fréquence pondérale des granules sphéroïdaux d'un échantillon de sel, dont le diamètre est égal à $d_i$ (G. HERDAN - "Small particle statistics" - 2nd edition - 1960 - Butterworths - pages 10 et 11), les diamètres $d_i$ étant mesurés par tamisage selon la norme AFNOR.

Les granules sphéroïdaux constituant le sel selon l'invention se particularisent par un diamètre moyen supérieur à 0,6 mm et inférieur à 3 mm. On a observé que la sélection, conformément à l'invention, de granules sphéroïdaux de diamètre moyen supérieur à 0,6 mm a pour effet de réduire considérablement la tendance à l'agglutination du sel selon l'invention. Par ailleurs, la sélection, conformément à l'invention, d'un diamètre moyen inférieur à 3 mm est une condition impérative pour l'obtention d'une teneur en chlorure de sodium supérieure à 99,5 en poids. On a en effet observé qu'en production industrielle, dès que le diamètre moyen du sel égale ou excède 3 mm, il devient très difficile de garantir, de manière reproductible, une teneur en eau inférieure à 0,5 % en poids, même au moyen d'un séchage intensif.

On préfère, selon l'invention, sélectionner un diamètre moyen au moins égal à 1 mm, de préférence à 1,5 mm.

Selon une forme de réalisation avantageuse de l'invention, le sel comprend moins de 50 ppm d'ions bromure, moins de 25 ppm d'ions phosphate, moins de 250 ppm d'ions sulfate, moins de 1 ppm d'arsenic, moins de 20 ppm de fer, moins de 100 ppm de calcium, moins de 10 ppm de plomb, moins de 500 ppm de potassium et moins de 0,2 ppm d'aluminium. Le sel selon cette forme de réalisation de l'invention présente la particularité intéressante d'être conforme aux pharmacopées européenne et des Etats-Unis.

Selon une autre forme de réalisation du sel selon l'invention, le diamètre moyen des granules sphéroïdaux est au moins égal à 1 mm (de préférence à 1,5 mm) et au maximum égal à 2,5 mm (de préférence à 2,2 mm). Le sel selon cette forme de réalisation de l'invention réalise une combinaison optimum de la résistance à l'agglutination et de la teneur en chlorure de sodium.

Il s'est révélé recommandable que les granules sphéroïdaux du sel selon l'invention présentent un étalement granulométrique le plus étroit possible. En pratique, selon une forme de réalisation recommandée de l'invention, les granules sphéroïdaux du sel selon l'invention présentent un étalement granulométrique de 0,9 mm à 5 mm, de préférence de 0,9 mm à 3,2 mm. Par définition, un étalement granulométrique de 0,9 mm à 5 mm (respectivement 3,2 mm) signifie que le diamètre des particules individuelles est au moins égal à 0,9 mm et au maximum égal à 5 mm (respectivement 3,2 mm). En d'autres termes, le sel selon cette forme de réalisation avantageuse de l'invention se trouve sensiblement exempt de particules de diamètre inférieur à 0,9 mm, et de particules de diamètre supérieur à 5 mm (respectivement 3,2 mm).

Selon une forme de réalisation supplémentaire du sel selon l'invention, que l'on préfère, les granules sphéroïdaux sont monolithiques.

On entend désigner par granule monolithique, un granule solide unitaire, non aggloméré, par opposition aux granules solides constitués d'agrégats ou d'agglomérats de particules.

Dans cette forme de réalisation préférée du sel selon l'invention, les granules sphéroïdaux sont avantageusement des cristaux. Bien qu'ils puissent être des monocristaux, on préfère que les granules sphéroïdaux du sel selon l'invention soient des polycristaux ou comprennent une majorité (par exemple plus de 50 % en poids) de polycristaux.

Le sel selon l'invention permet de respecter les normes de pureté imposées par les pharmacopées nationales et internationales telles que la pharmacopée européenne et la pharmacopée des Etats-Unis.

L'invention concerne dès lors également l'utilisation du sel selon l'invention en tant que sel pharmaceutique.

On entend désigner par sel pharmaceutique, du sel dont la teneur en chlorure de sodium et la pureté sont conformes aux pharmacopées et qui est dès lors susceptible d'être utilisé dans des applications ou des préparations pharmaceutiques sans nécessiter une épuration. Un exemple d'application pharmaceutique du sel selon l'invention est la préparation de solutions aqueuses de chlorure de sodium destinées à la dialyse rénale.

Le sel selon l'invention peut être obtenu par cristallisation au départ d'une solution aqueuse de chlorure de sodium. Avant la cristallisation, la solution aqueuse de chlorure de sodium doit généralement être soumise à une épuration réglée, en fonction du degré de pureté recherché pour le sel à produire. L'épuration de la solution aqueuse de chlorure de sodium doit être suffisante pour que le sel solide produit au départ de cette solution aqueuse présente une teneur pondérale en chlorure de sodium supérieure à 99 %, l'épuration devant prendre en compte la présence inévitable d'eau occluse dans les granules sphéroïdaux du sel.

Du sel conforme à l'invention, qui s'est révélé particulièrement avantageux est obtenu par mise en circulation d'une solution sursaturée de chlorure de sodium à travers un lit mobile de cristaux de chlorure de sodium, le lit mobile étant de préférence un lit fluidisé selon la définition généralement admise (GIVAUDON, MASSOT, et BENSIMONT - "Précis de génie chimique" - Tome 1 - 1960 - Berger-Levrault, Nancy - pages 353 à 370). Des particularités et détails concernant cette technique de mise en oeuvre du sel selon l'invention se trouvent décrits et revendiqués dans la demande de brevet EP-A-0352847 (SOLVAY & CIE).

L'intérêt de l'invention va apparaître à la lecture des exemples qui suivent.

Dans les exemples dont la description suit, on a comparé le comportement au stockage d'échantillons de sel conformes à l'invention, d'une part, et d'échantillons de sel de qualité pharmaceutique, non conformes à l'invention, d'autre part. Les échantillons de sel conformes à l'invention ont été obtenus par cristallisation au départ d'une solution aqueuse sursaturée de chlorure de sodium, au moyen du procédé décrit dans la demande de brevet EP-A-0352847 (SOLVAY & CIE).

Première série d'essais (Exemples 1 à 5).

Exemples 1 à 4 (conformes à l'invention).

Dans ces exemples, on a préparé du sel conforme à l'invention au moyen du procédé décrit dans la demande de brevet EP-A-0352847 (SOLVAY & CIE), consistant à faire circuler une solution sursaturée de chlorure de sodium de bas en haut à travers un lit fluidisé de cristaux de chlorure de sodium. On a prélevé quatre échantillons de 50 kg du sel ainsi produit et on les a soumis à un test de résistance à la prise en masse au cours du stockage et à une mesure de la variation de la teneur en eau du sel sous l'effet du stockage.

Le tableau I ci-dessous reproduit la distribution granulométrique des quatre échantillons de sel, mesurée par tamisage selon la norme AFNOR.

Tableau I

| Exemple n° | 1 | 2 | 3 | 4 |
|---|---|---|---|---|
| Refus à 3,55 mm | 0 % | 0 % | 0 % | 1,1 % |
| Refus à 3,15 mm | 0 % | 0,1 % | 0 % | 14,8 % |

Tableau I   (suite)

| Exemple n° | 1 | 2 | 3 | 4 |
|---|---|---|---|---|
| Refus à 2,8 mm | 20,4 % | 18,6 % | 5,8 % | 38,7 % |
| Refus à 2,5 mm | 41,7 % | 39,9 % | 20,2 % | 54,5 % |
| Refus à 2,0 mm | 76,9 % | 71,0 % | 56,1 % | 77,6 % |
| Refus à 1,6 mm | 88,2 % | 86,6 % | 72,8 % | 87,8 % |
| Refus à 1,4 mm | 93,1 % | 92,5 % | 81,6 % | 91,9 % |
| Refus à 1,25 mm | 96,7 % | 96,0 % | 89,1 % | 94,7 % |
| Refus à 1,0 mm | 98,5 % | 98,6 % | 95,8 % | 96,5 % |
| Diamètre moyen (mm) | 2,4 | 2,35 | 2,1 | 2,6 |

Pour déterminer la résistance à la prise en masse des échantillons de sel et la variation de leur teneur en eau, on a enfermé chaque échantillon de sel dans un sac étanche en polyéthylène, que l'on a ensuite entreposé. L'emballage des échantillons de sel dans les sacs a été opéré dans les conditions suivantes :

| température du sel | 53 °C; |
|---|---|
| température ambiante | 20,5 °C; |
| humidité relative | 67 %; |
| durée de l'emballage | 1 heure. |

Les sacs ont été entreposés sur le sol, dans un magasin industriel, pendant 14 jours, dans les conditions atmosphériques suivantes :

| température | 14-32 °C; |
|---|---|
| humidité relative | 50-80 %. |

A l'issue du stockage, les sacs ont été ouverts et on a mesuré, sur chaque échantillon, le taux de prise en masse, la variation de la coulabilité avant et après l'entreposage en sacs et la variation de la teneur en eau avant et après l'entreposage en sacs, ces paramètres étant définis ci-dessous.
Pour mesurer le taux de prise en masse du sel, on a déversé le contenu du sac sur une grille calibrée, disposée à une distance définie sous le sac, et on a mesuré le poids relatif des agglomérats de sel qui ont été arrêtés par la grille.
La coulabilité exprime la vitesse d'écoulement du sel à travers une ouverture calibrée, sous l'effet de la gravité.
Pour mesurer la teneur en eau du sel, on a broyé celui-ci à l'état de fines particules et on a ensuite mesuré de manière classique le degré d'humidité de l'échantillon broyé.
Les résultats sont mentionnés dans le tableau II ci-dessous.

Tableau II

| Exemple n° | 1 | 2 | 3 | 4 |
|---|---|---|---|---|
| Taux de prise en masse (%) | 0 | 0 | 0 | 0 |
| Coulabilité (g/s) | | | | |
| . avant entreposage | 19,9 | 20,5 | 21,2 | 19,1 |
| . après entreposage | 20,4 | 21,0 | 22,6 | 20,4 |
| Teneur en eau (g/kg) | | | | |
| . avant entreposage | 1,68 | 2,71 | 0,88 | 0,86 |
| . après entreposage | 0,77 | 0,89 | 0,22 | 0,22 |

Exemple 5 (de référence)

On a réalisé, dans les mêmes conditions qu'aux exemples 1 à 4, un essai avec du sel de qualité pharmaceutique, acquis en pharmacie. La distribution granulométrique du sel soumis à l'essai est reproduite au tableau III et les résultats de l'essai sont mentionnés au tableau IV.

Tableau III

| Refus à 630 µm | 0,3 % |
|---|---|
| Refus à 500 µm | 15,1 % |
| Refus à 400 µm | 36,8 % |
| Refus à 315 µm | 63,9 % |
| Refus à 250 µm | 81,5 % |
| Refus à 200 µm | 89,8 % |
| Refus à 160 µm | 94,7 % |
| Refus à 125 µm | 97,2 % |
| Refus à 80 µm | 99,3 % |
| Diamètre moyen | 360 µm |

Tableau IV

| Taux de prise en masse (%) | 36 |
|---|---|
| Coulabilité (g/s) | |
| . avant entreposage | 3,7 |
| . après entreposage | 0 |
| Teneur en eau (g/kg) | |
| . avant entreposage | 0,19 |
| . après entreposage | 0,11 |

<u>Seconde série d'essais</u> (Exemples 6 à 10)

<u>Exemples 6 à 9</u> (conformes à l'invention).

En exploitant le procédé selon l'invention, on a préparé un second lot de quatre échantillons de sel de 50 kg et on a examiné leur comportement au stockage en mesurant, pour chacun d'eux, les paramètres définis plus haut aux exemples 1 à 5.

Le tableau V ci-dessous reproduit la distribution granulométrique des quatre échantillons de sel, mesurée par tamisage selon la norme AFNOR.

Tableau V

| Exemple n° | 6 | 7 | 8 | 9 |
|---|---|---|---|---|
| Refus à 3,55 mm | 0 % | 0 % | 0 % | 0 % |
| Refus à 3,15 mm | 0,1 % | 0 % | 0 % | 0,2 % |
| Refus à 2,8 mm | 8,3 % | 2,9 % | 0,5 % | 7,8 % |
| Refus à 2,5 mm | 27,2 % | 20,9 % | 7,2 % | 24,0 % |
| Refus à 2,0 mm | 80,2 % | 76,5 % | 69,2 % | 66,5 % |
| Refus à 1,6 mm | 98,2 % | 97,7 % | 97,2 % | 85,0 % |
| Refus à 1,4 mm | 99,6 % | 99,7 % | 99,6 % | 89,5 % |
| Refus à 1,25 mm | 99,7 % | 99,9 % | 99,8 % | 92,1 % |
| Refus à 1,0 mm | 99,8 % | 99,9 % | 99,8 % | 94,3 % |
| Diamètre moyen (mm) | 2,3 | 2,25 | 2,1 | 2,25 |

L'emballage des échantillons de sel dans les sacs en polyéthylène a été opéré dans les conditions suivantes :

| température du sel | 39 °C; |
|---|---|
| température ambiante | 11,0 °C; |
| humidité relative | 31 %; |

(suite)

| durée de l'emballage | 1 heure. |
|---|---|

Les sacs ont été entreposés sur le sol, dans un magasin industriel, pendant 29 jours, dans les conditions atmosphériques suivantes :

| température | 8-20 °C; |
|---|---|
| humidité relative | 60-90 %. |

Les résultats sont mentionnés dans le tableau VI ci-dessous.

Tableau VI

| Exemple n° | 6 | 7 | 8 | 9 |
|---|---|---|---|---|
| Taux de prise en masse (%) | 0 | 0 | 0 | 0 |
| Coulabilité (g/s) | | | | |
| . avant entreposage | 19,4 | 20,3 | 21,1 | 20,7 |
| . après entreposage | 19,8 | 21,8 | 22,0 | 21,3 |
| Teneur en eau (g/kg) | | | | |
| . avant entreposage | 0,63 | 0,39 | 1,52 | 0,65 |
| . après entreposage | 0,79 | 0,38 | 0,83 | 0,98 |

Exemple 10 (de référence)

On a réalisé, dans les mêmes conditions qu'aux exemples 6 à 9, un essai avec du sel de qualité pharmaceutique, acquis en pharmacie. La distribution granulométrique du sel soumis à l'essai est reproduite au tableau VII et les résultats de l'essai sont mentionnés au tableau VIII.

Tableau VII

| Refus à 630 μm | 2,5 % |
|---|---|
| Refus à 500 μm | 24,8 % |
| Refus à 400 μm | 46,5 % |
| Refus à 315 μm | 70,8 % |
| Refus à 250 μm | 85,5 % |
| Refus à 200 μm | 92,4 % |
| Refus à 160 μm | 95,4 % |
| Refus à 125 μm | 97,5 % |
| Refus à 80 μm | 99,3 % |
| Diamètre moyen | 390 μm |

Tableau VIII

| Taux de prise en masse (%) | 70 |
|---|---|
| Coulabilité (g/s) | |
| . avant entreposage | 3,8 |
| . après entreposage | 0 |
| Teneur en eau (g/kg) | |
| . avant entreposage | 0,051 |
| . après entreposage | 0,065 |

Une comparaison des résultats des exemples 1 à 4 et 6 à 9 (concernant du sel conforme à l'invention) avec ceux des exemples 5 et 10 (concernant du sel de qualité pharmaceutique accessible en pharmacie, non conforme à l'invention), montre que le sel selon l'invention présente un comportement au stockage nettement supérieur à celui du sel non conforme à l'invention.

Troisième série d'essais

Les quatre exemples dont la description suit servent à démontrer le caractère critique de la sélection, conformément à l'invention, du diamètre moyen des granules sphéroïdaux du sel selon l'invention, en fonction du degré de pureté recherché, en particulier de la teneur en eau du sel. Dans ces quatre exemples, les échantillons de sel ont été obtenus par cristallisation au départ d'une solution aqueuse sursaturée de chlorure de sodium au moyen du procédé décrit et revendiqué dans la demande de brevet EP-A-0352847 (SOLVAY & CIE).

Exemple 11

Dans cet exemple, on a préparé un échantillon de sel conforme à l'invention, à l'état de granules de diamètre moyen égal à 2,4 mm. Pour déterminer la teneur en eau de l'échantillon de sel, on l'a broyé à l'état d'une fine poudre et on a mesuré le taux d'humidité de la poudre : 0,44 % en poids.

Exemple 12

On a répété l'essai de l'exemple 11, avec un échantillon de sel conforme à l'invention de diamètre moyen égal à 2,1 mm. On a mesuré, sur cet échantillon de sel, une teneur pondérale en eau égale à 0,32 %.

Exemple 13

On a répété l'essai de l'exemple 11, avec un échantillon de sel non conforme à l'invention de diamètre moyen égal à 3,05 mm. On a mesuré la teneur en eau de cet échantillon de sel, de la même manière qu'aux exemples 11 et 12. On a trouvé une valeur de 0,87 %.

Exemple 14

On a répété l'essai de l'exemple 11 avec un échantillon de sel non conforme à l'invention, à l'état de granules de diamètre moyen égal à 4,05 mm. On a mesuré, sur cet échantillon de sel, une teneur pondérale en eau, égale à 1,68 %.

Une comparaison des résultats obtenus aux exemples 11 à 14 montre que la teneur en eau du sel est une fonction croissante du diamètre moyen des granules sphéroïdaux du sel. On observe en particulier que, dès que le diamètre moyen des granules sphéroïdaux du sel excède 3 mm (Exemples 13 et 14), la teneur pondérale en eau du sel excède 0,5 %, de sorte qu'il est impossible d'obtenir une teneur pondérale en chlorure de sodium supérieure à 99,5 % et, dès lors, de respecter la norme NF T90-612 relative au sel pour la régénération des résines échangeuses d'ions.

**Revendications**

1.   Sel comprenant plus de 99.5% en poids de chlorure de sodium, **caractérisé en ce qu'**il se trouve à l'état de granules sphéroïdaux de diamètre moyen supérieur à 0,6 mm et inférieure à 3 mm et présente une teneur pondérale en eau inférieure à 0,5 %.

2.   Sel selon la revendication 1, dans lequel les granules sphéroïdaux ont un diamètre moyen de 1,5 à 2,2 mm.

3.   Sel selon la revendication 1 ou 2, dans lequel les granules sphéroïdaux présentent un étalement granulométrique de 0,9 mm à 3,2 mm.

4.   Sel selon l'une quelconque des revendications 1 à 3, dans lequel les granules sphéroïdaux présentent un rapport axial moyen au moins égal à 0,5, défini par la relation :

$$\frac{\Sigma^{i=n} a_i:b_i}{n}$$

où $a_i$ et $b_i$ désignent respectivement la plus petite et la plus grande dimension axiale d'un granule sphéroïdal, et n désigne le nombre de granules sphéroïdaux d'un échantillon de granules sphéroïdaux.

5. Sel selon l'une quelconque des revendications 1 à 4, dans lequel les granules sphéroïdaux sont des blocs monolithiques cristallins.

6. Sel selon la revendication 5, pouvant être obtenu par mise en circulation d'une solution sursaturée de chlorure de sodium, de bas en haut à travers un lit fluidisé de cristaux de chlorure de sodium.

7. Sel selon l'une quelconque des revendications 1 à 6, comprenant, en valeurs pondérales, moins de 50 ppm d'ions bromure, moins de 25 ppm d'ions phosphate, moins de 250 ppm d'ions sulfate, moins de 1 ppm d'arsenic, moins de 20 ppm de fer, moins de 100 ppm de calcium, moins de 10 ppm de plomb, moins de 500 ppm de potassium et moins de 0,2 ppm d'aluminium.

8. Utilisation du sel selon l'une quelconque des revendications 1 à 7, comme sel pharmaceutique.

**Patentansprüche**

1. Salz, umfassend mehr als 99,5 Gew.-% Natriumchlorid, **dadurch gekennzeichnet, dass** es sich in einem Zustand sphäroidaler Körnchen mit einem mittleren Durchmesser von größer als 0,6 mm und kleiner als 3 mm befindet und einen Feuchtigkeitsgehalt in Gewicht von kleiner als 0,5 % aufweist.

2. Salz nach Anspruch 1, worin die spheroidalen Körnchen einen mittleren Durchmesser von 1,5 bis 2,2 mm haben.

3. Salz nach Anspruch 1 oder 2, worin die spheroidalen Körnchen eine Korngrößenverteilung von 0,9 mm bis 3,2 mm aufweisen.

4. Salz nach einem der Ansprüche 1 bis 3, worin die spheroidalen Körnchen ein mittleres axiales Verhältnis von mindestens gleich 0,5 aufweisen, definiert durch die Beziehung:

$$\frac{\Sigma^{i=n} a_i:b_i}{n}$$

worin ai und bi die kleinere bzw. größere axiale Ausdehnung eines spheroidalen Körnchens bezeichnen und n die Anzahl der spheroidalen Körnchen einer Probe spheroidaler Körnchen bezeichnet.

5. Salz nach einem der Ansprüche 1 bis 4, worin die spheroidalen Körnchen kristalline monolitische Blöcke sind.

6. Salz nach Anspruch 5, dadurch erhältlich, dass man eine gesättigte Natriumchloridlösung von unten nach oben quer durch eine Wirbelschicht aus Natriumchloridkristallen in Zirkulation versetzt.

7. Salz nach einem der Ansprüche 1 bis 6, umfassend, in Gewichtswerten, weniger als 50 ppm Bromionen, weniger als 25 ppm Phosphationen, weniger als 250 ppm Sulfationen, weniger als 1 ppm Arsen, weniger als 20 ppm Eisen, weniger als 100 ppm Calcium, weniger als 10 ppm Blei, weniger als 500 ppm Kalium und weniger als 0,2 ppm Aluminium.

8. Verwendung des Salzes nach einem der Ansprüche 1 bis 7 als pharmazeutisches Salz.

**Claims**

1. Salt comprising more than 99.5% by weight of sodium chloride, **characterized in that** it is in the form of spheroidal granules with a mean diameter of greater than 0.6 mm and of less than 3 mm and exhibits a content by weight of water of less than 0.5%.

2. Salt according to Claim 1, in which the spheroidal granules have a mean diameter of 1.5 to 2.2 mm.

3. Salt according to Claim 1 or 2, in which the spheroidal granules exhibit a particle size range from 0.9 mm to 3.2 mm.

4. Salt according to any one of Claims 1 to 3, in which the spheroidal granules exhibit a mean axial ratio at least equal to 0.5, defined by the relationship:

$$\frac{\Sigma^{i=n} a_i : b_i}{n}$$

where ai and bi respectively denote the smallest and the greatest axial dimension of a spheroidal granule and n denotes the number of spheroidal granules of a sample of spheroidal granules.

5. Salt according to any one of Claims 1 to 4, in which the spheroidal granules are crystalline monolithic blocks.

6. Salt according to Claim 5, which can be obtained by circulating a supersaturated sodium chloride solution from the bottom upwards through a fluidized bed of sodium chloride crystals.

7. Salt according to any one of Claims 1 to 6, comprising, in values by weight, less than 50 ppm of bromide ions, less than 25 ppm of phosphate ions, less than 250 ppm of sulphate ions, less than 1 ppm of arsenic, less than 20 ppm of iron, less than 100 ppm of calcium, less than 10 ppm of lead, less than 500 ppm of potassium and less than 0.2 ppm of aluminium.

8. The use of the salt according to any one of Claims 1 to 7 as pharmaceutical salt.